# EUROPEAN PATENT APPLICATION

(11) **EP 4 260 857 A1**
(43) Date of publication of application: **18.10.2023**
(21) Application number: 21903316.4
(22) Date of filing: 03.12.2021
(51) Int. Cl.: A61K 31/4439, A61K 8/49, A61P 17/14, A61Q 5/12, A61Q 7/00

(54) **COMPOSITION FOR HAIR IMPROVEMENT**

(30) Priority: 07.12.2020 JP 2020202460
(71) Applicant: Dr. Cherry Inc., Tokyo 160-0023 (JP)
(72) Inventor: FUKUOKA Hirotaro, Tokyo 160-0023 (JP)
(74) Representative: Hoffmann Eitle
(86) International application number: PCT/JP2021/044394
(87) International publication number: WO 2022/124212

(57) **Abstract**

The present invention provides a compound for improving (ameliorating) hair volume or quality, and a composition containing the compound. Specifically, according to the present invention, a composition containing pioglitazone for use in improving hair volume or quality is provided.

## Description

### [Technical Field]

The present invention relates to a composition for hair improvement.

### [Background Art]

A hair restorer through inhibition of androgens has been developed. This treatment exerts, however, a hair growth effect during the treatment, but the hair growth effect is lost when the treatment is ended. This suggests that the hair growth effect through the inhibition of androgens is symptomatic and is not a fundamental treatment through tissue regeneration.

It is described that deletion of PPARγ in the skin shows no phenotype, and that hair shifted to the anagen stage was not observed in the skin treated with a PPARγ antagonist (Non-Patent Literature 1).

### [Citation List]

### [Non-Patent Literature]

[Non-Patent Literature 1] Festa E. et al., Cell, 146: 761-771, 2011

### [Summary of Invention]

The present invention provides a composition for hair improvement.

The present inventors have found that pioglitazone can be used in improving hair volume or quality. The present invention is based on this finding.

The present invention provides the following inventions:
(1) A composition containing pioglitazone, for use in improving hair volume or quality.
(2) The composition according to (1) described above, being a composition for oral administration.
(3) The composition according to (1) described above, being a composition for topical administration to the skin.

(11) A composition containing pioglitazone, for use in improving hair volume or quality by accelerating tissue repair of the skin accompanying increase of an M2 macrophage and increase of PPARγ expression.
(12) A composition containing pioglitazone, for use in increasing a hair thickness.
(13) A composition containing pioglitazone, for use in adjusting or improving hair cuticle alignment.
(14) The composition according to any one of (11) to (13) described above, being a composition for oral administration.
(15) The composition according to any one of (11) to (13) described above, being a composition for topical administration to the skin.
(16) The composition described above being a pharmaceutical.

(20) A composition containing pioglitazone, for use in improving hair volume or quality by accelerating tissue repair of the skin accompanying increase of an M2 macrophage and increase of PPARγ expression.
(21) A composition containing pioglitazone, for use in increasing anagen-stage hairs.
(22) A composition containing pioglitazone, for use in increasing a hair thickness.
(23) A composition containing pioglitazone, for use in improving hair cuticle quality (for example, adjusting or improving the alignment of hair cuticle).
(24) A method for increasing hair volume or quality (preferably, a hair thickness or hair cuticle quality) in a subject, including administering pioglitazone to the subject.
(25) The method according to (24) described above, in which the administration is topical administration to the skin of the subject.
(26) The method according to (24) described above, in which the administration is oral administration to the subject.
(27) The method according to any one of (24) to (26) described above, further including, after the administration of pioglitazone, testing the hair volume or quality of the subject.
(28) The method according to any one of (24) to (27) described above, further including, after the administration of pioglitazone, testing whether or not tissue repair of the skin accompanying an increase of M2 macrophage and increase of PPARγ expression on the skin or scalp has been accelerated.
(29) A composition containing pioglitazone, for use in the method according to any one of (24) to (28) described above.
(30) Use of pioglitazone in the production of a composition for use in the method according to any one of (24) to (28) described above.
(31) The composition that is a pharmaceutical or for pharmaceutical use.
(32) The composition that is a cosmetic or for cosmetic use.

### [Brief Description of Drawings]

[Figure 1] Figure 1 is a diagram illustrating the change in thickness of hair growing from the scalp treated with pioglitazone. An upper panel illustrates the difference in the hair thickness between a treated side and an untreated side when only a half side of the scalp is treated. In graphs, "#1" indicates a result obtained 1 month after the treatment, and "#6" indicates a result obtained 6 months after the treatment. A lower panel indicates a comparison between a change rate to the thickness obtained on the untreated side, and a change rate to the thickness obtained on the treated side.
[Figure 2] Figure 2 illustrates electron microphotographs of hair cuticle in a portion of the scalp treated with pioglitazone and a portion of the scalp not treated.
[Figure 3] Figure 3 illustrates the results of hair quality evaluation based on hair cuticle in the portion of the scalp treated with pioglitazone and the portion of the scalp not treated.
[Figure 4] Figure 4 illustrates an effect of the pioglitazone treatment on expression of specific genes.
[Figure 5] Figure 5 illustrates an effect of the pioglitazone treatment on expression of specific genes in the human scalp.

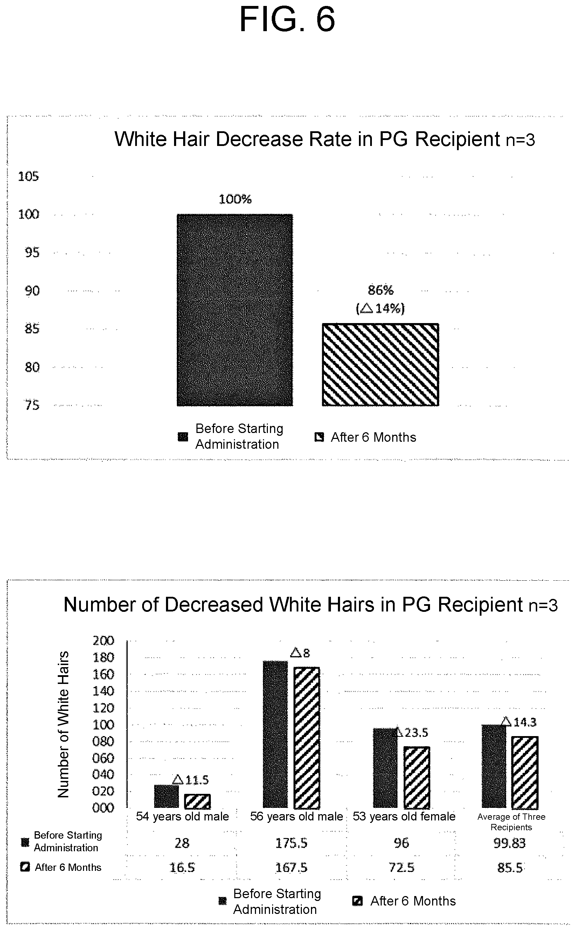

[Figure 6] Figure 6 illustrates data on the decrease of the number of white hairs obtained in an example.
[Figure 7A] Figure 7A is a schematic diagram of a method for dividing the scalp into nine evaluation regions employed in an example.
[Figure 7B] Figure 7B illustrates a degree of progression of hair thinning before the treatment with pioglitazone, and a degree of progression of hair thinning after the treatment with pioglitazone.

### [Description of Embodiment]

Herein, a "subject" can be a mammal, and examples include pets such as a dog, a cat, a rabbit, a hamster, a guinea pig, a rat, and a squirrel; domestic animals such as a cow, a pig, a horse, a sheep, and a goat; and primates such as a monkey, a chimpanzee, an orangutan, a gorilla, a bonobo, and a human. The subject can be one suffering or not suffering from diabetes, and preferably can be a non-diabetes patient.

Herein, a "treatment" is used in the meaning encompassing a therapeutic treatment and a preventive treatment. Herein, therapy can be used in the meaning encompassing suppression of aggravation of a disease or a condition, delay of aggravation of a disease or a condition, improvement of a disease or a condition, or cure of a disease or a condition. Herein, prevention can be used in the meaning encompassing suppression of onset of a disease or a condition, or delay of onset of a disease or a condition.

A "pharmaceutical" used herein means a product used with expectation of a clinical effect such as a therapeutic effect or a preventive effect. Examples of the pharmaceutical include a pharmaceutical composition and a transplantation material. A "dermatologic composition" used herein means a composition suitable for topical application to the skin. Examples of the dermatologic composition include a pharmaceutical suitable for topical application to the skin, and a cosmetic composition. Examples of the cosmetic composition include personal care compositions topically applied to the skin, such as a moisturizer, a conditioner, an antiaging agent, a whitening agent, a sunscreen agent, an antiperspirant, a shaving composition, an aftershave composition, a foundation, a lip balm, a lipstick, a hair conditioner, soap, shampoo, a cleaner, and a lubricant. Examples of a personal care product include, in addition to the personal care compositions, structures not in the form of a composition (such as underwear, diapers, tissue paper, a wiping cloth, a mask, and a patch).

Pioglitazone is a thiazolidine-based oral hypoglycemic agent. Pioglitazone has a structure represented by the following Chemical Formula (I):

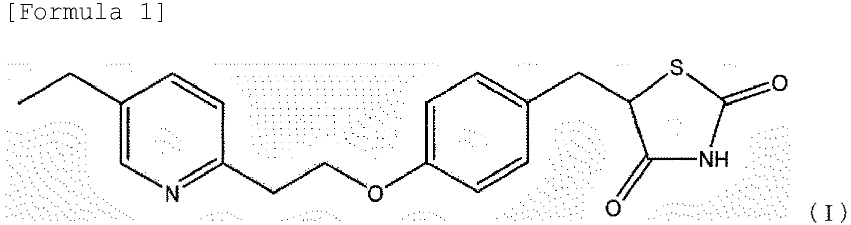

According to the present invention, pioglitazone can improve the volume or quality of hair (for example, hair of the head). More specifically, pioglitazone increased the volume of hair (for example, the volume of anagen-stage hair), aligned hair cuticle, decreased the number or rate of white hairs, and/or improved (ameliorated) a hair thickness (and hair gloss and bound, and hair elasticity and shininess). Accordingly, the present invention provides a composition containing pioglitazone for use in improving hair quality (for example, a hair thickness, hair gloss and bounce, or hair elasticity and shininess). Regarding the hair thickness, the number or rate of hairs having a diameter of 40 µm or more, a diameter of 50 µm or more, a diameter of 60 µm or more, a diameter of preferably 70 µm or more, a diameter of more preferably 80 µm or more, and a diameter of further preferably 90 µm or more can be increased, and the composition of the present invention can be used for this use. This effect is different from that of an anti-androgen agent (such as Finasteride) that increases hairs having a diameter of 40 µm. The hair thickness can be increased, as compared with that before the treatment, by 5% or more, by 6% or more, by 7% or more, by 8% or more, by 9% or more, or by 10% or more, and the composition of the present invention can be used for this use. In one aspect, the present invention provides a composition containing pioglitazone for use in aligning hair cuticle (adjusting or improving alignment of cuticle). In another aspect, the present invention provides a composition containing pioglitazone for use in reducing the number or rate of white hairs. In another aspect, the present invention provides a composition containing pioglitazone for use in increasing a hair thickness (and hair gloss and bounce).

In the present invention, the subject can be a subject having white hair. Besides, in the present invention, the subject can be a subject having thinning hair. The subject having thinning hair can be, for example, a subject having alopecia (such as androgenetic alopecia (AGA)). In the present invention, the subject can be a subject having hair with disturbed (peeled, or roughened) cuticle. Besides, in the present invention, the subject can be a subject having hair with a thickness (diameter) of 70 µm or less, 60 µm or less, 50 µm or less, or 40 µm or less. In the present invention, the subject can be a subject having experienced a treatment with an anti-androgen agent (for example, a subject having AGA). In the present invention, the subject can be a subject that has not experienced progress arrest or improvement of hair thinning even through treatment with an anti-androgen agent (for example, a subject having AGA) .

In the present invention, pioglitazone can be orally administered to the subject. Therefore, in the present invention, the composition containing pioglitazone can be a composition for oral administration. The composition for oral administration can be, for example, a powder, a granule, an oral tablet, a capsule, or a pill. Pioglitazone can be a pharmaceutically acceptable salt thereof, such as pioglitazone hydrochloride.

In the present invention, pioglitazone can be topically administered to the skin of the subject. Therefore, in the present invention, the composition containing pioglitazone can be a dermatologic composition or a pharmaceutical composition for topical administration to the skin. The pharmaceutical composition for topical administration to the skin can be in a dosage form suitable for an administration form such as transdermal administration, intradermal administration or subcutaneous administration. The dermatologic composition can be in a dosage form suitable for transdermal administration. The dermatologic composition, or the pharmaceutical composition for topical administration to the skin may be transdermally increased in penetration through the skin by osmosis technique such as electroporation. For example, in order to increase penetration, an active ingredient can be penetrated through the skin with META-TDS Electroporation Machine (CTP-802, Grand Aespio Inc., Korea) at an output of 3 V/6 mA {actually measured value} with the subject caused to grasp an earth wrapped with wet gauze with his/her one hand. In this aspect, the pharmaceutical composition can contain suspended pioglitazone. In another aspect, the pharmaceutical composition can contain dissolved pioglitazone. In one aspect, the pharmaceutical composition can be a suspension containing pioglitazone. In one aspect, the pharmaceutical composition can be an emulsion, a cream, or an oil containing pioglitazone.

The composition of the present invention can be a composition such as a personal care composition or a pharmaceutical composition.

An example of the pharmaceutical composition includes a pharmaceutical composition for topical administration, which can be used in the present invention. The pharmaceutical composition for topical administration can be a pharmaceutical composition for mucosal administration or body surface administration, and examples include eye drops, an eye ointment, a sublingual tablet, a buccal tablet, a lozenge, a mouthwash, a spray, an aerosol, and an inhalant; a solution formulation such as a liquid, an irrigant, a glycerin formulation, a spirit, a liquor, or a salve; a dispersion formulation such as an emulsion, a suspension, a liniment, a lotion, a spray, or a liposomal agent; a semi-solid formulation such as an ointment, a plaster, a patch, an adhesive tape agent, a dermatological paste agent, a poultice, a cream agent, an oil agent, or a stick agent; and an exudation formulation such as an extract (a soft extract or a dry extract), and a tincture.

In the present invention, the pharmaceutical composition may contain a pharmaceutically acceptable excipient. Examples of the pharmaceutically acceptable excipient include a solvent, a base, a diluent, an extending agent, a filler, and a vehicle; a dissolution assisting agent, a solubilizing agent, a buffer, a tonicity agent, an emulsifier, a suspending agent, a dispersant, a thickener, a gelling agent, a curing agent, an absorber, an adhesive agent, an elastomer, a plasticizer, a sustained release agent, and a propellant; and an antioxidant, a preservative, a moisturizer, a shading agent, an antistatic agent, an aromatic, a flavor, a colorant, and an emollient.

Examples of the personal care composition include a skin care product, an antiperspirant, a deodorant, a beauty product, cosmetics, and a hair care product. Examples of the personal care composition include a moisturizer, a conditioner, an antiaging agent, a whitening agent, a sunscreen agent, an antiperspirant, a shaving composition, an aftershave composition, a foundation, a lip balm, a lipstick, a hair conditioner, shampoo, a cleaner, and a lubricant. The personal care composition can be used in a personal care product. Examples of the personal care product include underwear, diapers, tissue paper, a wiping cloth, a mask, and a patch. The composition may contain an excipient in addition to the active ingredient. The composition can be formed into a dosage form suitable for an administration form such as intravenous administration, transdermal administration, oral administration, enteral administration, and intraperitoneal administration. For preventing and/or treating dermatitis, the composition of the present invention may be administered by transdermal administration, and the composition can be in the form of, for example, a gel, an emulsion, a cream, a liquid, a paste, a lotion, or a liposome cream (for example, a dermatologic composition). In one aspect, the composition can be an ointment. In transdermal administration, a dermatologically acceptable excipient can be used as the excipient, and a dosage form suitable for this administration can be employed. In transmucosal administration, an excipient acceptable for mucosal administration can be used as the excipient, and a dosage form suitable for this administration can be employed.

The present invention provides a method for improving hair quality in a subject in need thereof, including administering pioglitazone to the subject. In the present invention, the administration can be topical administration. In the present invention, the administration is performed on the scalp in need of improvement of hair quality.

The present invention provides pioglitazone for improving hair quality. The present invention provides use of pioglitazone in the manufacture of a pharmaceutical, a cosmetic, or a personal care product for use in improvement of hair quality.

In one aspect of the present invention, the composition of the present invention may use together with another pharmaceutical, cosmetic or composition for modifying the scalp or skin. Another pharmaceutical, cosmetic, or composition can be:
(i) A pharmaceutical composition for injection containing a secretion from an adipose stem cell as an active ingredient, for use in inducing tissue regeneration in a scalp tissue or skin tissue, the secretion being obtained by culturing the adipose stem cell in a serum-free medium under hypoxic condition {wherein 0.3 to 0.5 µg, or 0.35 to 0.45 µg, in terms of protein amount, of the secretion is preferably subcutaneously administered or intradermally administered per section of the scalp or skin};
(ii) a pharmaceutical composition for injection containing a secretion from an adipose stem cell as an active ingredient, for use in treating a wound on body surface, the secretion being obtained by culturing the adipose stem cell in a serum-free medium under hypoxic condition {wherein 0.3 to 0.5 µg, or 0.35 to 0.45 µg, in terms of protein amount, of the secretion is preferably subcutaneously administered or intradermally administered per section of the scalp or a body surface tissue around a wound site};
(iii) a pharmaceutical composition for injection containing a secretion from an adipose stem cell as an active ingredient, for use in modifying hair, the secretion being obtained by culturing the adipose stem cell in a serum-free medium under hypoxic condition {wherein 0.3 to 0.5 µg, or 0.35 to 0.45 µg, in terms of protein amount, of the secretion is preferably subcutaneously administered or intradermally administered per section of the scalp or skin}; or
(iv) (a) a pharmaceutical composition for topical administration for use in modifying the scalp or skin, (b) a pharmaceutical composition for topical administration for use in treating a wound, or (c) a pharmaceutical composition for topical administration for use in accelerating hair growth or a pharmaceutical composition for topical administration for use in modifying hair, containing a therapeutically effective amount of saturated fatty acid, or a pharmaceutically acceptable salt thereof {wherein the pharmaceutical composition may contain saturated fatty acid and another additive, and the saturated fatty acid can be one or more fatty acids selected from the group consisting of palmitic acid, stearic acid, and myristic acid}.

These details are as disclosed in US 2020/0101114A and US2020/0246409A, the entire of which are incorporated herein by reference. In the present invention, pioglitazone can be administered to a subject having been treated with any one or more of the above-described (i) to (iv).

### [Examples]

### Example 1: Change in Hair thickness

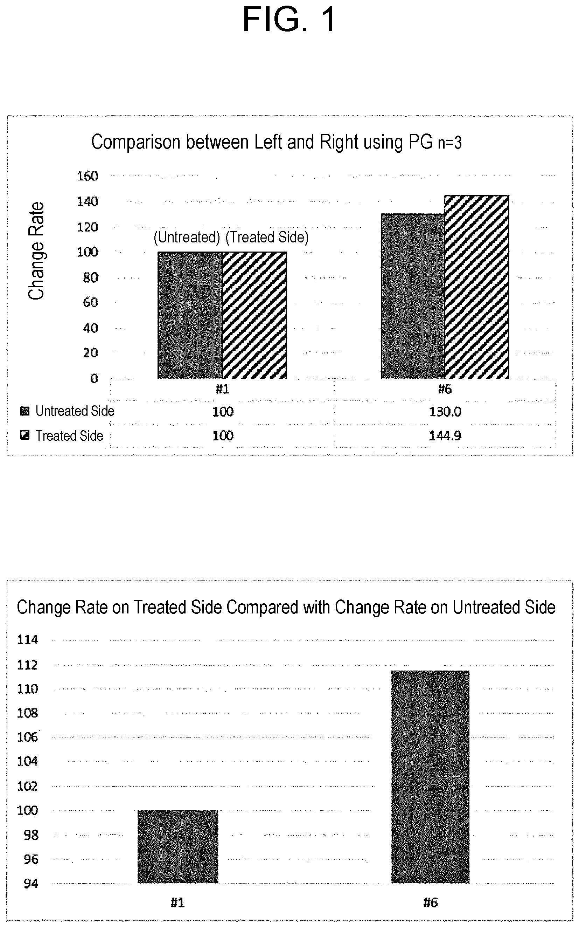

As pioglitazone, a 15 mg pioglitazone tablet "NP" was used. One pioglitazone tablet was ground in a mortar, and the resultant was suspended in 3 mL of water for injection PL "FUSO" for use. Subjects were three female patients (69, 48, and 65 years old patients). In each of the patients, lines were extended from both outer canthi toward the head, two points on these lines crossing a line extended between the both ears through the vertex were tattooed with an India ink, and a portion around the marked center was photographed under the same conditions. A suspension of pioglitazone was applied to the entire of half side of the scalp. In order to increase penetration, pioglitazone was caused to penetrate through the skin with META-TDS, Electroporation Machine (CTP-802, Grand Aespio Inc., Korea) at an output of 3 V/6 mA {actually measured value}. This treatment was performed at a frequency of once every two weeks 12 times (namely, for 6 months). The test sites were away from the midline of the head by 51 mm on average on both left and right sides. After 6 months from the start of the treatment (after performing the treatment 12 times), Dermo Prime, dpharris (CHOWIS, Korea) was used to import data of two images of each test site into a computer as digital images, and in each image, three hairs having the largest thickness (6 hairs in two images of each test site) were selected to automatically measure the thickness. An average value of the tested values (hair thickness: mm) was used for graphing. Specifically, assuming that the thickness before the treatment was 100, the thickness after the treatment was indicated as a rate. Regarding a patient group in which the treatment was performed on a half side, a difference between before and after the treatment and a difference between the left and right-side portions were compared. As illustrated in Figure 1, in the comparison between the left and right side portions, it was confirmed that the hair was increased in the thickness, as compared with that of a negative control, on the pioglitazone-treated side (PG treated side) as the number of times of the treatment increased from 1 month after the treatment (after #1) to 6 months after the treatment (after #6). A lower panel of Figure 1 indicates a change rate obtained on the treated side to a change rate obtained on the untreated side. As illustrated in the lower panel of Figure 1, it was confirmed that the change rate obtained on the PG treated side was higher than the change rate obtained in the negative control.

According to the results illustrated in the upper panel of Figure 1, the state that the hair thickness increased with progress of the treatment was found even under the negative control (administration of water for injection PL). Considering that the change of the hair thickness was not observed in the patient over several months before starting the treatment, this was presumed that the therapeutic effect was propagated from the PG administered side (treated side) to the untreated side.

Therefore, as a negative control, saline was injected to one half side of the head (with the other half side untreated). Change in hair (the number of hairs having a length of 1.4 mm or more) caused by the injection of saline to the one half side of the head every month for 6 months was observed, a change rate between before the treatment and 6 months after treatment was -0.84% to + 1.24%, and the average was 1.03%. Besides, change caused on 6 months after treatment between the treated side having saline injected and the untreated side (with nothing injected) was -0.99% to +1.25%, and the average was 1.06% (n = 4). The change in hair (a total number of hairs having a length of 1.4 mm or more) caused by the injection of saline to the one half side of the head every month for 6 months was observed while the change between before the treatment and 6 months after treatment was -0.79% to +1.33%, and the average was 1.05%. Besides, change caused on 6 months after treatment between the treated side having saline injected and the untreated side was -0.95% to +1.28%, and the average was 1.07% (n = 4). In this manner, in the group administered with saline as the negative control, a significant increase of hairs having a length of 1.4 mm or more was not found on day 3 after shaving. The thickness of the hair grown by less than 1.4 mm on day 3 after shaving was 75.39 µm on average. There was a trend that the thickness of the hair grown by 1.4 mm or more was 97.42 µm or more on average. Hair having a diameter of 80 µm can be visually identified to be sufficiently thick.

In this manner, in the group administered with saline instead of PG, an improving effect in the hair thickness was not found.

It was revealed, based on these results, that modification of the scalp with pioglitazone leads to improvement of a growing rate of hair (particularly, anagen-stage hair), and increase of thick hairs having bounce and resilience. Besides, the hair thickness improving effect of the hair thickness was found on the untreated side in the half side treatment of the scalp, while this was suggested as propagation of the scalp modifying effect from the treated side.

### Example 2: Change in Hair Quality

In this example, it was investigated whether or not it is observed any improvement of hair cuticle before and after the treatment.

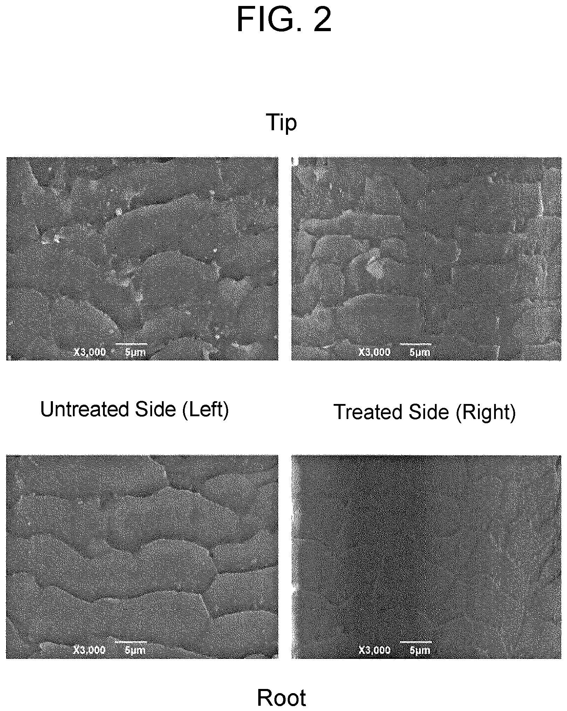

The hair of the group administered with PG (administered twice a month) of Example 3 was collected, and in consideration of the growing rate (15 mm/month) of the hair of the patient, a portion corresponding to a period 2 months to 3 months (about 2.5 months) before the treatment and a portion corresponding to a period 4 months to 5 months (about 4.5 months) after starting the treatment were collected to be hermetically stored respectively in Ziploc(R). Thereafter, hair of a test site was adhesively fixed on a fixing plate with a resin. The resultant was hermetically stored, and on test day, was subjected to carbon fixation, and a state of hair cuticle was observed by an ordinary method with a scanning electron microscope on the same day. As a negative control, hair of the same patient in a portion not treated (untreated in the half side treatment) was used. Then, a portion of the hair newly grown (on the root side) and an existing portion (on the tip side) were compared with each other. A representative example is illustrated in Figure 2.

As a result, it was observed, in the treated hair, that while the cuticle was rough on the tip side, the cuticle was aligned on the root side corresponding to the newly grown portion, and thus, the hair quality was obviously improved. On the contrary, in the hair of the untreated portion of the negative control, although a root portion was highly rated, cuticle roughness was observed also on the tip side, and thus, an effect of improving the quality was not found in the untreated portion.

The same evaluation was performed on a larger number of patients (three patients: females: 48, 65 and 69 years old) (15 mg of PG, administered twice a month, administration 12 times in total, 6 months). The evaluation was performed 20 days after the final administration based on an electron microscope image in accordance with the following rating. The evaluation was performed by 6 doctors. An average of ratings was obtained.

### List of Ratings of Hair Quality Evaluation

5: Cuticle is aligned, and is not rough at all.
4: Cuticle is aligned than average, but is slightly rough.
3: Cuticle is aligned averagely.
2: Cuticle is rough than average, and is slightly peeled.
1: Cuticle is rough as a whole, and is peeled as a whole.

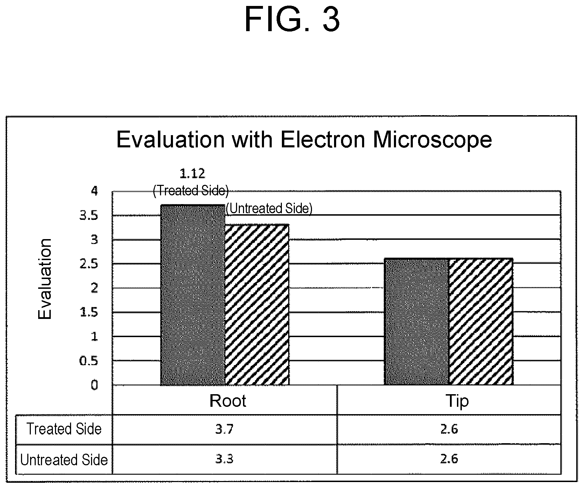

Results are shown in Table 1 and Figure 3.

### [Table 1]

**Table 1: Change in Hair Quality in Treated Site and Untreated Site**

| | Root | Tip |
|---|---|---|
| Treated Side | 3.7 | 2.6 |
| Untreated Side | 3.3 | 2.6 |
| Degree of Improvement | 1.12 | 1.0 |

As described above, the hair quality was changed after the administration in the PG treated group. This reveals that pioglitazone leads to an effect of improving hair quality.

### Example 3: Change in Gene Expression in Tissue after Subcutaneous Administration

Next, change in gene expression in a tissue was checked. As a subject, 12 weeks old Wistar Rat (male) was used. Regarding a 2.5 µg/portion administration group, three regions were set on the back from the head to the tail, and each of these regions was divided into two regions on both left and right sides of the spine to set six regions on the back in total. In such an administration group, PG was administered to two portions around the center of each of the six regions. Specifically, in the 2.5 µg/portion administration group (n = 3), 2.5 µg (20 µL) of PG was administered per portion. Besides, regarding a 0.1 µg/portion administration group and a 0.02 µg/portion administration group, two regions were set from the head to the tail, and each of these regions was divided into two regions on both left and right sides of the spine, and thus, four regions in total were set on the back. In such an administration group, PG was administered to two portions around the center of each of the four regions. Specifically, in the 0.1 µg/portion administration group (n = 3), 0.1 µg (20 µL) of PG was administered per portion, and in the 0.02 µg/portion administration group (n = 2), 0.02 µg (20 µL) of PG was administered per portion. It is noted that a distance between the two portions was 1 cm. After 8 weeks, a tissue was collected with a 3 mm punch after shaving a portion on the center of the injected portion.

The collected tissue piece was frozen with liquid nitrogen, and stored at -80°C. Purification and extraction of a total RNA from the stored tissue piece were performed by enclosing 25 mg of the tissue piece together with 5 stainless steel beads (diameter: 3 mm) in a sample tube to homogenize the tissue piece with RNeasy Mini Kit (QIAGEN GmbH, Hilden, Germany) and Tissue Lyser II (QIAGEN GmbH, Hilden, Germany), and then by using a spin column in accordance with protocol attached to the kit. For gene expression analysis by quantitative PCR, design and synthesis of a gene specific primer and a probe were entrusted to Integrated DNA Technologies, Inc. (Skokie, IL, USA), and an expression level of a target gene was analyzed with Prime Time Gene Expression Master Mix reagent (Integrated DNA Technologies, Inc., Skokie, IL, USA) and a Rotor-Gene Q PCR device (QIAGEN GmbH, Hilden, Germany). The PCR analysis was performed twice or three times per gene of each sample.

Information of seven kinds of rat genes used in the gene expression analysis by quantitative PCR (qPCR), and nucleotide sequences of a probe and a primer set synthesized for each gene are as follows:
1.) Ribosomal protein lateral stalk subunit P0 (RplP0), Rattus norvegicus: mRNA, 1,093 bp, NM_022402.2, GI: 310616731
   Probe: 6-FAM/CCT GTC TTC /ZEN/CCT GGG CAT CAC G/3IABkFQ (SEQ ID No. 1)
   Primer-1: CAA TCC CTG ACG CAC CG (SEQ ID No. 2)
   Primer-2: TGT CTG CTC CCA CAA TGA AG (SEQ ID No. 3)
2.) CD34, Rattus norvegicus: CD34 molecule (Cd34), mRNA 1,161 bp NM_001107202.2 GI: 169790781
   Probe: 6-FAM/TCC CTG GAA /ZEN/GTA CCA GCC ACT ACT /3IABkFQ (SEQ ID No. 4)
   Primer-1: GGA GTA TTT CCA CCA GTT CCT AC (SEQ ID No. 5)
   Primer-2: GAT GGC TGG TGT GGT CTT ATT (SEQ ID No. 6)
3.) Peroxisome proliferator-activated receptor gamma transcript variant 1, (PPARG), Rattus norvegicus: mRNA 1,805 bp NM_013124.3 GI: 223941861
   Probe: 6-FAM/CCT GGG CGG /ZEN/TCT CCA CTG AGA ATA /3IABkFQ (SEQ ID No. 7)
   Primer-1: GAA TTA GAT GAC AGT GAC TTG GC (SEQ ID No. 8)
   Primer-2: AGC AGG TTG TCT TGG ATG TC (SEQ ID No. 9)
4.) CD163 molecule (Cd163), Rattus norvegicus: mRNA 4,223 bp NM_001107887.1 GI:157823808
   Probe: 6-FAM/ACC CAA CGG /ZEN/CTT ACA GTT TCC TCA A/3IABkFQ/ (SEQ ID No. 10)
   Primer-1: TCA TCC GTC TTC GAA TCC ATC (SEQ ID No. 11)
   Primer-2: TCT CAT TGC CTT CCT CTT GTG (SEQ ID No. 12)
5.) CD68 molecule (Cd68), Rattus norvegicus: mRNA 1,224 bp NM_001031638.1 GI: 72255506
   Probe: 6-FAM/CTC TGA TGT /ZEN/CGG TCC TGT TTG AAT CCA /3IABkFQ/ (SEQ ID No. 13)
   Primer-1: TGA GAA TGT CCA CTG TGC TG (SEQ ID No. 14)
   Primer-2: CAT TCC CTT ACG GAC AGC TTA C (SEQ ID No. 15)

A difference (ΔCt), in an expression level of mRNA, between ribosomal protein lateral stalk subunit P0 (RplP0) of a housekeeping gene and the target gene (average of Ct obtained from the qPCR reaction of each gene, average of 2 tests of each sample of 3 rats) was calculated, and in addition, a difference (ΔΔCt) of ΔCt between the experimental sample to be analyzed and a control (with no PG administration) was calculated. This value of ΔΔCt was converted into a relative gene expression ratio (rate) of the target gene, and the rate was plotted on a graph.

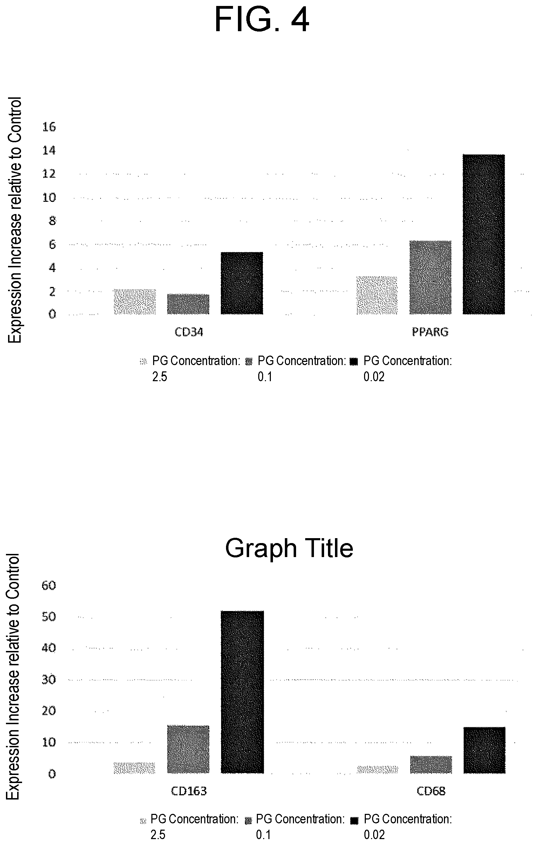

Results are as illustrated in Figure 4. As illustrated in Figure 4, the expression of CD34 and PPARγ was found to increase in the pioglitazone administration group. Besides, as illustrated in Figure 4, CD68 and CD163 were found to simultaneously increase in the tissue of the pioglitazone administration group. This seems to indicate that activity of an M2 macrophage is higher than that of an M1 macrophage in the tissue of the pioglitazone administration group, namely, suggests that tissue repair is accelerated in the pioglitazone administration group (see Festa et al., Cell, 146: 761-771, 2011, the entire of which is incorporated herein by reference). Besides, the increase of expression of CD34 and PPAPγ suggests that an adipose stem cell and a juvenile adipocyte (preadipocyte) were induced in the skin. Festa et al., 2011 discloses that expression of PPARγ is induced in a preadipocyte, and describes that the preadipocyte is increased at the start of the anagen stage. Festa et al., 2011 also discloses that an Azip mouse lacking in a mature adipocyte has a preadipocyte, and regenerates hair follicle similarly to a wild type mouse. In this manner, the expression of CD34 and PPARγ suggests that the anagen stage in hair cycle has started.

A human subject was also checked. A suspension of pioglitazone was prepared in the same manner as in Example 2, and the whole amount of the suspension was applied to the head of a human subject (42 years old male). The PG treatment was performed on the whole scalp three times at a frequency of once every two months. A tissue around the test site was collected with a 3 mm punch before the treatment and 2 months after performing the treatment three times (6 months after the first treatment). The collected tissue was halved, and one half was fixed with 10% formalin, and stored under refrigeration. The other half was immersed in RNA Stabilization Solution (RNAlater, Thermo Fisher Scientific, Lithuania), and the resultant was frozen in liquid nitrogen, and stored at -80°C. Thereafter, the gene expression analysis was performed in the same manner as the rat gene expression analysis.

A difference (ΔCt), in an expression level of mRNA, between ribosomal protein lateral stalk subunit P0 (RplP0) of a housekeeping gene and the target gene (average of Ct obtained from the qPCR reaction of each gene performed three times) was calculated, and in addition, a difference (ΔΔCt) in ΔCt between samples of the subject to be analyzed obtained before and after the treatment and a control (two persons not treated) was calculated. Based on the value of ΔΔCt thus obtained, a relative expression ratio (change in gene expression caused before and after the treatment) of the target gene was obtained, and plotted on a graph.

Probes and primers used in the quantitative PCR were as follows:
1)Homo sapiens ribosomal protein lateral stalk subunit P0 (RPLP0), transcript variant 1, mRNA, 1,105 bp
   Accession: NM_001002.4 GI: 1519314286
   Probe: 56-FAM/CCC TGT CTT /ZEN/CCC TGG GCA TCA C/3IABkFQ/ (SEQ ID No. 16)
   Primer-1: TGT CTG CTC CCA CAA TGA AAC (SEQ ID No. 17)
   Primer-2: TCG TCT TTA AAC CCT GCG TG (SEQ ID No. 18)
2)Homo sapiens CD68 molecule (CD68), transcript variant 2, mRNA 1,790 bp Accession: NM_001040059.1 GI: 91199549
   Probe: 56-FAM/AGG TCC TGC /ZEN/ATG AAT CCA AAG CTG A/3IABkFQ/ (SEQ ID No. 19)
   Primer-1: CCA TGT AGC TCA GGT AGA CAA C (SEQ ID No. 20)
   Primer-2: CCA CCT GCT TCT CTC ATT CC (SEQ ID No. 21)
3)Homo sapiens CD163 molecule (CD163), transcript variant 1, mRNA 4,190 bp Accession: NM_004244.5 GI: 344179109
   Probe: 56-FAM/ATC CAT CTG /ZEN/AGC AGG TCA CTC CAG/3IABkFQ/ (SEQ ID No. 22)
   Primer-1: CCA GAA CAC ATA TTC CCT CCA C (SEQ ID No. 23)
   Primer-2: GCA TAG TAA CTG TAC TCA CCA ACA (SEQ ID No. 24)
4)Homo sapiens CD34 molecule (CD34), transcript variant 1, mRNA 2,621 bp Accession: NM_001025109.1 GI: 68342037
   Probe: 56-FAM/CCT TGC AAC /ZEN/ATC TCC CAC TAA ACC CT/3IABkFQ (SEQ ID No. 25)
   Primer-1: TGC CTG AAC ATT TGA TTT CTG C (SEQ ID No. 26)
   Primer-2: GAC CTT TCA ACC ACT AGC ACT (SEQ ID No. 27)

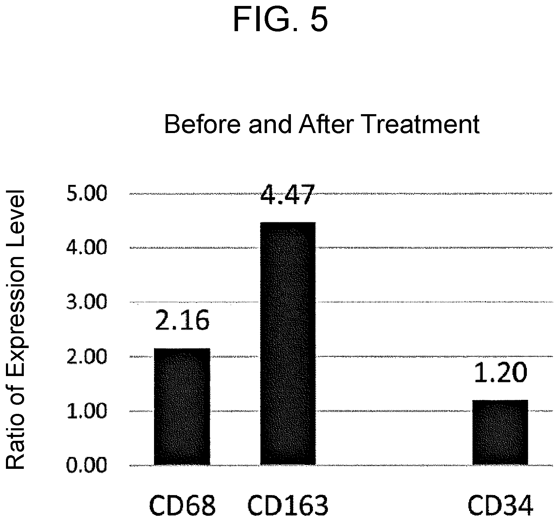

Results are as illustrated in Figure 5. In the tissue of the patient after the administration of pioglitazone, CD68 and CD163 were also found to increase in the same manner as in the rat. This seems to indicate that activity of an M2 macrophage is higher than that of an M1 macrophage in the tissue after the treatment with pioglitazone, namely, suggests that tissue repair is accelerated in the pioglitazone administration group.

### Example 4: Change in White Hair

As pioglitazone, a 15 mg pioglitazone tablet "NP" was used. Each patient took one tablet containing 15 mg of pioglitazone around 10 o'clock in the morning once every day. Subjects were four patients (two males: 54 and 56 years old, two females: 52 and 53 years old). In each of the patients, lines were extended from both outer canthi toward the head, two points on these lines crossing a line extended between the both ears through the vertex were tattooed with an India ink, and a portion around the marked center was photographed under the same conditions.

The 53 years old male patient had no white hair in a specific portion at the start, a temporal portion on the line extending between the both ears was tattooed with an Indian ink, and a portion around the marked center was photographed under the same conditions.

Pioglitazone was taken continuously for 6 months, and the same test was performed every two months.

For trichogram, a portion around the marked center was photographed (Canon Powder Shot A520, Tokyo, Japan), and hair within a circle having a diameter of 11 mm (area: 95 mm²) around the tattooed portion set as a center in an imaging range was photographed in two directions, and the number was visually counted to obtain an average of the two photographs.

As a result, in the three patients having white hair before the treatment, the white hair was decreased in the above-mentioned shaved portion in all of the three patients by taking pioglitazone for 6 months. Specifically, it was observed that white hair of 12.7 hairs/95 mm² on average was decreased in the above-mentioned shaved portion (n = 3). Assuming that the number of white hairs was 100 before the administration, the number of white hairs was decreased by 23.2% (n = 3).

In this example, the effect of improving the hair quality by pioglitazone was shown. It was suggested based on the gene expression data that pioglitazone induces tissue repair, and it was suggested that the effect of improving hair quality is caused as a result of the tissue repair by pioglitazone.

### Example 5: Scalp Improvement Effect in Each Site

In this example, the scalp was divided into nine regions to observe the effect of the treatment therein. In this manner, even when each patient has both a good portion (portion having a large hair volume) and a poor portion (portion having a small hair volume), influence on the good portion and influence on the poor portion can be separately evaluated, and thus, it can be expected that highly accurate evaluation can be realized.

Subjects were four androgenetic alopecia (AGA) male patients (of 42 to 63 years old) having aggravated as compared with a state 1 year ago. Two patients out of these patients had continuously taken Finasteride, an anti-androgen agent, respectively from 4 years ago and 7 years ago, and continuously took it after starting the following treatment. In this example, pioglitazone, that is a PPARy agonist, was orally administered to these patients. Specifically, a pioglitazone tablet containing 15 mg of pioglitazone (Nihon Generic Co., Ltd.; JG, 120 mg/tablet) was ground in a mortar, and 40 mg of the obtained powder (having a pioglitazone content of 5 mg) was mixed with lactose (260 mg) to obtain a single pack. The single pack was orally administered to each patient together with drinking water in the morning once a day. A photograph of each patient taken from directly above 10 to 12 months before starting the treatment of this example, a photograph taken on the day of starting the treatment, and a photograph taken after 2 months from the start of the treatment were evaluated. The evaluation was performed by 5 medical specialists in charge of treatment of hair thinning for outpatients.

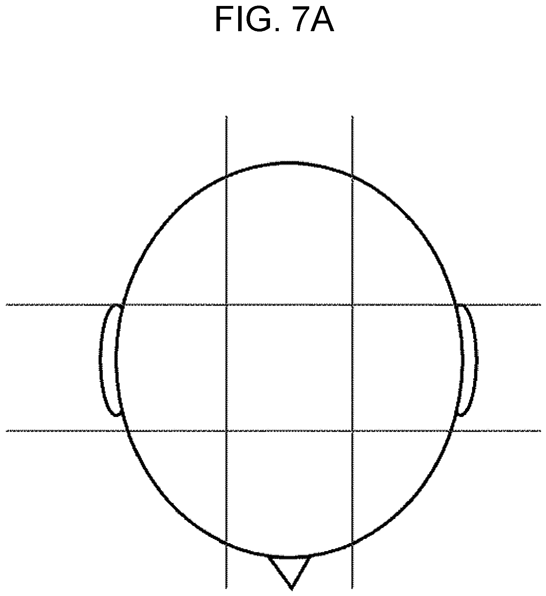

The nine regions were set based on the following rule: Specifically, a head image photographed from the top of the head was assumed as an ellipse, the major axis was equally divided into three sections, and the minor axis was equally divided into three sections, so as to separate the ellipse into nine regions. Figure 7A is a schematic diagram for illustrating how the nine sections of the head are divided. A hair growth effect was evaluated by the 5 medical specialists in accordance with the following rating. A see-through degree was obtained based on how much the scalp was seen through as compared with normal scalp when the head was observed from the top of the head.

### Rating of Hair growth Effect

5: substantially normal
4: see-through degree of 20% or less
3: see-through degree of 40% or less
2: see-through degree of 60% or less
1: see-through degree of 80% or less

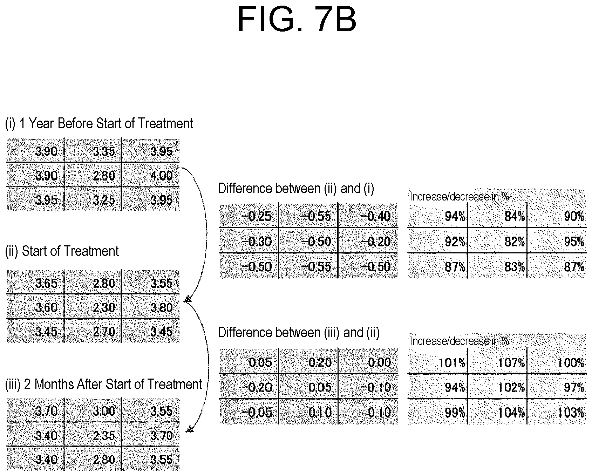

Results were as illustrated in Figure 7B. Figure 7B illustrates an average of ratings of each section of the divided nine regions. Before starting the treatment, it was observed that symptoms of hair thinning were aggravated in all the patients. On the contrary, it could be understood that when pioglitazone was started to be orally taken, the progress of hair thinning was at least stopped even in a short period of 2 months (see Figure 7B). In particular, trends toward improvement were observed in a site having a high degree of aggravation. In this manner, it was revealed, in this example, that the state of the patient in trends toward aggravation at the start of the treatment was in trends toward improvement. This reveals that a PPARγ agonist is effective in the treatment of hair thinning. In particular, the PPARγ agonist increases a juvenile adipocyte (preadipocyte) to shift the scalp to the anagen stage, while it was presumed that the effect was exhibited on hair thinning in AGA also owing to this mechanism.
[Sequence Listing] [Final] PF30-0009 Sequence Listing ST25.txt

## Claims

1. A composition comprising pioglitazone, for use in improving hair volume or quality, and increasing anagen-stage hairs.

2. The composition according to claim 1, being a composition for oral administration.

3. The composition according to claim 1, being a composition for topical administration to a skin.

4. A composition comprising pioglitazone, for use in inducing a juvenile adipocyte, or inducing histogenesis of a skin accompanying induction of a juvenile adipocyte.

5. The composition according to claim 4, wherein the juvenile adipocyte includes a CD34-positive and PPARγ-positive adipocyte.

6. A method for improving hair volume or quality in a subject, comprising:
administering pioglitazone to the subject.

7. The method according to claim 6, wherein the administration is topical administration to a skin of the subject.

8. The method according to claim 6, wherein the administration is oral administration to the subject.

9. The method according to any one of claims 6 to 8, further comprising, after the administration of pioglitazone, testing the hair volume or quality of the subject.

10. The method according to any one of claims 6 to 9, further comprising, after the administration of pioglitazone, testing whether or not tissue repair of the skin accompanying increase of M2 macrophage and increase of PPARγ expression on the skin or scalp has been accelerated.

11. The method according to claim 10, further comprising administering pioglitazone to the subject in which tissue repair of a skin accompanying increase of M2 macrophage and increase of PPARγ expression on the skin or scalp has been accelerated after the administration of pioglitazone.

12. A composition comprising pioglitazone, for use in the method according to any one of claims 6 to 11.

13. Use of pioglitazone in the manufacture of a composition for use in the method according to any one of claims 6 to 11.
